(19) Europäisches Patentamt
European Patent Office
Office européen des brevets

(11) Veröffentlichungsnummer : **0 277 089 B1**

(12) # EUROPÄISCHE PATENTSCHRIFT

(45) Veröffentlichungstag der Patentschrift : **12.06.91 Patentblatt 91/24**

(21) Anmeldenummer : **88730017.6**

(22) Anmeldetag : **22.01.88**

(51) Int. Cl.$^5$ : **C07J 1/00,** C07J 5/00, C07J 7/00, A61K 31/565, A61K 31/57, C07J 17/00, C07J 21/00, C07J 41/00

(54) **11 Beta-alkinyl-estrene und -estradiene, deren Herstellung und diese enthaltende pharmezeutische Präparate.**

(30) Priorität : **23.01.87 DE 3702383**

(43) Veröffentlichungstag der Anmeldung : **03.08.88 Patentblatt 88/31**

(45) Bekanntmachung des Hinweises auf die Patenterteilung : **12.06.91 Patentblatt 91/24**

(84) Benannte Vertragsstaaten : **AT BE CH DE ES FR GB GR IT LI LU NL SE**

(56) Entgegenhaltungen :
**EP-A- 0 156 284**
**DE-A- 2 805 490**
**FR-A- 2 377 418**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Ottow, Eckhard, Dr.**
**Sonnenallee 124**
**W-1000 Berlin 44 (DE)**
Erfinder : **Wiechert, Rudolf, Prof. Dr.**
**Petzower Strasse 8a**
**W-1000 Berlin 39 (DE)**
Erfinder : **Neef, Günter, Dr.**
**Darmstädter Strasse 9**
**W-1000 Berlin 15 (DE)**
Erfinder : **Bardenhagen, Jürgen, Dr.**
**Im Baumgarten 1**
**W-7818 Vogtsburg/Oberbergen (DE)**
Erfinder : **Beier, Sybille, Dr.**
**Uhlandstrasse 121**
**W-1000 Berlin 31 (DE)**
Erfinder : **Elger, Walter, Dr.**
**Schorlemer Allee 12B**
**W-1000 Berlin 33 (DE)**
Erfinder : **Henderson, David, Dr.**
**Jahnstrasse 17**
**W-1000 Berlin 28 (DE)**

**EP 0 277 089 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft den in den Patentansprüchen gekennzeichneten Gegenstand, d.h. neue 11β-substituierte Steroide, Verfahren zu ihrer Herstellung, diese Verbindungen enthaltende pharmazeutische Präparate und ihre Verwendung zur Herstellung von Arzneimittteln.

Die erfindungsgemäßen Verbindungen werden durch die allgemeine Formel I beschrieben

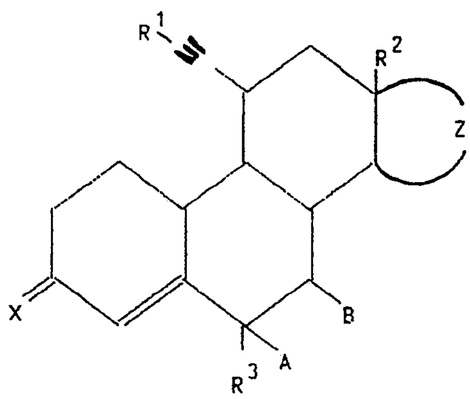

(I),

worin

A und B gemeinsam für eine zweite Bindung zwischen den Kohlenstoffatomen 6 und 7 oder jeweils für ein Wasserstoffatom,

X für ein Sauerstoffatom, zwei Wasserstoffatome oder die Hydroxyiminogruppierung N ~OH,

Z für den Rest eines Rings der Formel

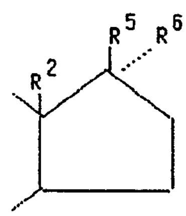

worin

| | |
|---|---|
| $R^5/R^6$ | $-OR^7/-C\equiv C-Y$ |
| | $-OR^7/-\underset{\overset{\|}{O}}{C}-CH_2-R^8$ |
| | $-\underset{\overset{\|}{O}}{C}-CH_2-R^8/-OR^7$ |
| | $-\underset{\overset{\|}{O}}{C}-CH_2-R^8/-CH_3$ |
| | $-\underset{\overset{\|}{O}}{C}-CH_2-R^8/-H$ |
| | $-OR^7/-(CH_2)_m-CH_2-R^9$ |
| | $-OR^7/-CH=CH(CH_2)_k-CH_2-R^9$ |
| | $-OR^{10}/-H$ |

oder einen Rest der Formel

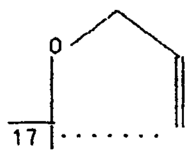

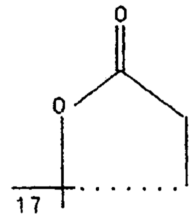

bezeichnet, mit

$R^7$ in der Bedeutung eines Wasserstoffatoms oder Acylrestes mit 1 bis 4 Kohlenstoffatomen, Y in der Bedeutung eines Wasserstoffatoms, einer Alkyl-, Hydroxyalkyl-, Alkoxyalkyl- oder Acyloxyalkylgruppe mit jeweils 1 bis 4 Kohlenstoffatomen im Alkyl- bzw. Acylrest,

$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,

$R^9$ in der Bedeutung eines Hydroxy- oder Cyanidrestes, einer O-Alkyl- oder O-Acylgruppe mit jeweils 1 bis 4 Kohlenstoffatomen, die gegebenenfalls substituiert ist durch eine $C_1$-$C_4$-O-Alkylgruppe,

$R^{10}$ in der Bedeutung eines Wasserstoffatoms, einer Alkyl- oder Acylgruppe mit jeweils 1 bis 10 Kohlenstoffatomen,

m in der Bedeutung 0, 1, 2 und 3,

k in der Bedeutung 0, 1 oder 2,

$R^1$ für einen Vinyl-oder Cyclo-1-alkenyl-Rest, eine Phenyl-, Naphthylgruppe oder einen 5-oder 6-gliedrigen Aromaten mit wenigstens einem Stickstoff-, Sauerstoff- oder Schwefelatom ; einen Vinyl-oder Cyclo-1-alkenylrest, eine Phenyl-, Naphthylgruppe oder einen 5- oder 6-gliedrigen Aromaten mit wenigstens einem Stickstoff-, Sauerstoff- oder Schwefelatom, substituiert durch 1-3 Halogenatome, 1-3 $C_1$-$C_4$-Alkylgruppen, eine $C_1$-$C_4$-O-Alkyl-, $C_2$-$C_{10}$-Alkenyl-, $C_1$-$C_4$-Acyl-, $C_1$-$C_4$-O-Acyl-, eine gegebenenfalls durch eine oder zwei $C_1$-$C_4$-Alkylgruppe(n) substituierte Aminogruppe, einen Phenyl, Nitro, Hydroxy-, Carboxy-, Cyanid oder $COOR^4$-Rest mit $R^4$ in der Bedeutung einer $C_1$-$C_4$-Alkylgruppe, die gegebenenfalls substituiert ist durch eine gegebenenfalls mit einem $C_1$-$C_4$-Alkyl-, $C_1$-$C_4$-O-Alkyl-, Halogen- oder Phenyl-Rest substituierte Phenylgruppe,

$R^2$ für eine Methyl- oder Ethylgruppe

$R^3$ für ein Wasserstoff-, Chloratom oder eine Methylgruppe stehen

Die in $R^1$, $R^5$ und $R^6$ bzw. $R^4$, $R^7$, $R^8$, $R^9$, $R^{10}$ und Y der allgemeinen Formel I enthaltenen Alkyl-, Alkoxy-, Acyl- sowie Acyloxygruppen sollen jeweils 1 bis 4 Kohlenstoffatome enthalten, wobei die Methyl-, Ethyl-, Propyl-, Isopropyl-, Methoxy, Ethoxy-, Propoxy-, Isopropoxy-, Formyl-, Acetyl-, Propionyl- und Isopropionylgruppe bevorzugt sind.

Von den Alkenylresten in $R^6$ sind die Propenyl- und Butenylgruppe, die in der E-oder Z-Konfiguration vorliegen können, bevorzugt, d.h. wenn $R^6$ für $-CH = CH-(CH_2)_k-CH_2-R^9$ steht, dann soll k bevorzugt Null oder 1 bedeuten. Als Alkenylreste in $R^1$ kommen gerad- und verzweigtkettige $C_2$-$C_{10}$-, bevorzugt $C_2$-$C_6$-Alkenylgruppen, wie beispielsweise Vinyl, 1-Methylvinyl, Propenyl, Butenyl oder Pentenyl infrage. Von den cyclischen Alkenylgruppen sind die Cyclo-1-penten- und Cyclo-1-hexen-gruppe bevorzugt. Als Halogen sind Chlor und Fluor bevorzugt.

Als weiterer Rest $R^1$ kommen sowohl substituierte wie auch unsubstituierte Phenyl-, 1-Naphthyl-, 2-Naphthyl- sowie 5- und 6-gliedrige heterocyclische Gruppen, die wenigstens 1 Stickstoff-, Sauerstoff- oder Schwefelatom enthalten, in Frage. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl und 2-Tetrazolyl.

Bei der Substitution des Aromaten ist die Monosubstitution bevorzugt.

Die neuen 11β-Acetylen-substituierten Steroide der allgemeinen Formel I werden erfindungsgemäß hergestellt durch eine bei 0 bis 100°C, vorzugsweise bei 20 bis 80°C, in polaren Lösungsmitteln unter der Einwirkung eines geeigneten (z.B. R.F. Heck, Palladium Reagents in Organic Syntheses Academic Press 1985, J. Organometal. Chem. 93, 253 [1975], J. Org. Chem. 31, 4071 [1966]) Kupplungs-Reagenzes durchgeführte Kupplungsreaktion eines 11β-Ethinyl-Steroids der allgemeinen Formel II

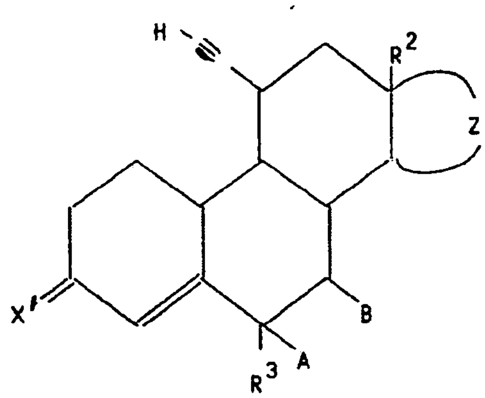

(II),

worin
A, B, $R^2$ und $R^3$ die oben genannte Bedeutung haben, X′ für ein Sauerstoffatom oder zwei Wassestoffatome steht und Z′ die gleiche Bedeutung wie Z hat, wobei jedoch in Z gegebenenfalls vorhandene acetylenische Wasserstoffatome in Z′ geschützt sind sowie in Z gegebenenfalls vorhandene O-Acylgruppen in Z′ als OH-Gruppen vorliegen und in Z oder Z′ gegebenenfalls vorhandene OH-Gruppen gewünschtenfalls geschützt sind,
mit einem Aryl-, Heteroaryl- oder Alkenylhalogenid der allgemeinen Formel III

$R^{1'}$-V (III),

worin

$R^{1'}$ die gleiche Bedeutung wie $R^1$ hat, wobei jedoch in $R^1$ gegebenenfalls vorhandene O-Acylgruppen in $R^{1'}$ als OH-Gruppen vorliegen und in $R^1$ oder $R^{1'}$ gegebenenfalls vorhandene OH-Gruppen gewünschtenfalls geschützt sind und

V für ein Chlor-, Brom- oder Jodatom steht.

Als geeignete Kupplungsreagenzien seien Salze bzw. Komplexe der Metalle Kupfer, Nickel oder Palladium oder deren Kombinationen genannt, die in äquimolaren bis katalytischen Mengen eingesetzt werden. Insbesondere sind zu nennen Kupferjodid, Bis(triphenylphosphin)nickel(II)chlorid, Tris(triphenylphosphin)nickel, Palladium(II)acetat, Bis(triphenylphosphin)palladium(II)-chlorid, Bis(triphenylphospin)palladium(II)acetat und Tetrakis(triphenylphospin)palladium.

Geeignete Lösungsmittel sind z.B. Dimethylformamid, Hexamethylphosphorsäuretriamid, Dimethylsulfoxid, sekundäre und tertiäre Amine wie Diäthylamin, Dipropylamin, Triäthylamin, sowie deren Gemische.

Die in der allgemeinen Formel II von Z′ umfaßten terminalen Acetylenschutzgruppen (zum Beispiel die Trimethylsilyl- oder tert.-Butyldimethylsilylgruppe) sind dem Fachmann bekannt und werden nach literaturbekannten Verfahren gespalten [Synthesis 1980, 627, J.Org.Chem. 46 (1986) 2280].

Die in den allgemeinen Formeln II und III Z′ und $R^{1'}$ umfaßten Hydroxy-Schutzgruppen sind im sauren Milieu leicht abspaltbare Gruppen, wie z.B. die Methoxymethyl-, Ethoxymethyl-, Methoxyethoxymethyl- oder Tetrahydropyranylgruppe.

Die freigesetzten Hydroxygruppen können in an sich bekannter Weise verestert oder verethert werden.

Die erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen −20 und +40°C in die Oxime (Formel I mit X in der Bedeutung der Hydroxyiminogruppierung N ~OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]nonen 5 (DBN) und 1,5-Diazabicyclo[5.4.0]undecen-5 (DBU), wobei Pyridin bevorzugt ist.

Die Herstellung der Ausgangsverbindungen der allgemeinen Formel II erfolgt durch Ausbildung der gewünschten Struktur des Ringes B, der Einführung des 11β-Ethinyl-Substituenten analog der Vorschrift in der deutschen Offenlegungsschrift DE-OS 28 05 490 bzw. in US-P 4,292,251 und der Variation des Ringes D bzw. des C-17-Substituentenmusters nach literaturbekannten Methoden, wobei die Reihenfolge der genannten Maßnahmen unterschiedlich sein kann.

Die Einführung der 6,7-Doppelbindung gelingt durch Allyl- oder Dienoletherbromierung und anschließende Bromwasserstoffabspaltung.

Die Allylbromierung wird zum Beispiel mit N-Bromsuccinimid, N-Bromacetamid, 1,3-Dibrom-5,5-dimethylhydantoin oder Dibromtetrachlorethan in Gegenwart eines Radikalbildners wie Dibenzoylperoxid in einem

Lösungsmittel vorgenommen. Als Lösungsmittel kommen aprotische Lösungsmittel wie Dioxan und chlorierte Kohlenwasserstoffe, wie zum Beispiel Tetrachlorkohlenstoff, Chloroform oder Tetrachlorethylen infrage. Die Umsetzung erfolgt zwischen 0°C und der Siedetemperatur der Lösung.

Die Dienoletherbromierung wird zum Beispiel analog der Vorschrift in Steroids I, 233 durchgeführt (siehe auch Fried, Edwards Organic Reactions in Steroid Chemistry).

Die Bromwasserstoffabspaltung unter Ausbildung der $\Delta^6$-Doppelbindung erfolgt durch Erhitzen der 6-Bromverbindung mit basischen Mitteln, vorzugsweise mit Lithiumbromid und Lithiumcarbonat oder mit Lithiumbromid und Calciumcarbonat in einem aprotischen Lösungsmittel wir Dimethylformamid bei Temperaturen von 50 bis 120°C. Eine weitere Möglichkeit der HBr-Abspaltung besteht darin, daß man die 6-Bromverbindung in Collidin oder Lutidin erhitzt.

Die Einführung des Chlor- bzw. Methyl-Substituenten in C-6 des Steroidgerüsts gelingt z.B. durch die in der Deutschen Auslegeschrift 1,158,966 bzw. in US-Patent 4,544,555 und US-Patent 4,196,203 angegebenen Methoden über die entsprechenden 6,7-Epoxide bzw. 6-Methylen-Derivate.

Die Entfernung der 3-Oxogruppe zu einem Endprodukt der allgemeinen Formel I mit X in der Bedeutung von zwei Wasserstoffatomen kann z.B. nach der in DOS 2805490 angegebenen Vorschrift durch Thioketalisierung und anschließende reduktive Spaltung erfolgen.

Die Einführung der Substituenten $R^5$ und $R^6$ erfolgt nach den üblichen Verfahren des C-17-Seitenketteaufbaus durch nucleophile Addition an das – durch z.B. Oppenauer-Oxidation der C-17-Hydroxygruppe erhaltene – 17-Keton und Folgereaktionen ("Terpenoids and Steroids", Specialist Periodical Report, The Chemical Society, London, Vol. 1-12)

Die nucleophile Addition von HC≡CY, in der Y eine Schutzgruppe wie zum Beispiel Trimethylsilyl oder tert.-Butyldimethylsilyl oder Alkyl mit 1 bis 4 C-Atomen bedeutet, erfolgt mit Hilfe einer Verbindung der allgemeinen Formel MC≡CY, in der Y die oben angegebene Bedeutung hat und M ein Alkalimetall darstellt.

Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die Einführung von 3-Hydroxypropin, -propen bzw. -propan in 17-Stellung erfolgt durch Umsetzung des 17-Ketons mit dem Dianion des Propargylalkohols (3-Hydroxypropin), zum Beispiel dem in situ generierten Dikaliumsalz des Propargylalkohols, zum 17α-(3-Hydroxyprop-1-inyl)-17β-hydroxyderivat oder mit metallierten Derivaten des 3-Hydroxypropins, zum Beispiel mit 1-Lithium-3-(tetrahydropyran-Z′-yloxy)-prop-1-in-1-id, zum 17-[3-(Tetrahydropyran-2′-yloxy)-prop-1-inyl]-17β-hydroxyderivat, die anschließend zu den 17-(3-Hydroxypropyl- bzw. Hydroxypropenyl)-17-hydroxy-Verbindungen hydriert werden können. Das gelingt zum Beispiel durch Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung der homologen Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards : Organic Reactions in Steroid Chemistry, Van Nostrand Reinhold Company 1972, Seite 134 ; und H.O. House : Modern Synthetic Reactions 1972, Seite 19). Als desaktivierte Edelmetallkatalysatoren kommen beispielsweise 10% Palladium auf Bariumsulfat in Gegenwart eines Amins oder 5% Palladium auf Calciumcarbonat unter Zusatz von Blei(II)-acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion dere acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid in flüssigem Ammoniak (j. Chem. Soc. 1955, 3558), mit Lithium in niederen molekularen Aminen (J. A. Chem. Soc. 77 (1955) 3378), mit Boranen (J. Am. Chem. Soc. 93 (1971) 3395 und 94 (1972) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxydationsstufe.

Die Einführung der Hydroxyalkene kann auch direkt erfolgen durch Addition einer entsprechenden metallierten Hydroxyalkenylverbindung, wie zum Beispiel 1-Lithium-3-(tetrahydropyran-2'-yloxy)-prop-1(E)-en (J. Org. Chem. 40 2265) oder 1-Lithium-3-(tetrahydropyran-2'-yloxy)-prop-1(Z)-en (Synthesis 1981, 999) Homologe können auf diese Art ebenfalls eingeführt werden.

Die Einführung von 3-Hydroxypropan in 17-Stellung kann ebenfalls direkt durch Umsetzung des 17-Ketons mit metallierten Derivaten von 3-Halogen-propanolen – wobei die Hydroxygruppe im Metallierungsschritt als Alkoholat (Tetrahedron Letters 1978, 3013) oder als geschützte Funktion (J. Org. Chem. 37, 1947) vorliegt – zu der 17-(3-Hydroxypropyl)-17β-hydroxy-verbindung bzw. zu der an der terminalen Hydroxygruppe geschützten Verbindung erfolgen. Als Schutzgruppen kommmen zum Beispiel die Ethoxyethyl-, Tetrahydropyranyl- und Methoxymethyl-Gruppen in Frage.

Werden Endprodukte der Formel I gewünscht mit $R^5/R^6$ in der Bedeutung von

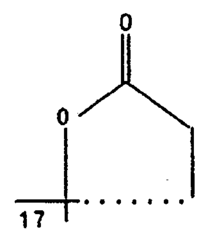

so wird die 17-(3-Hydroxypropyl)-Verbindung in an sich bekannter Weise oxydiert, zum Beispiel mit Jones' Reagenz, Braunstein, Pyridiniumdichromat, Pyridiniumchlorochromat, Chromsäure-Pyridin oder dem Fetizon-Reagenz Silbercarbonat Celite (Compt. rend. 267 [1968] 900).

Die Darstellung von Endprodukten der Formel I mit $R^5/R^6$ in der Bedeutung von

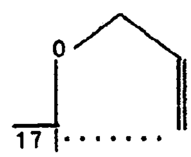

erfolgt (s. auch unten Beispiel 7) durch Ringschlußreaktion des entsprechenden 17-(3-Hydroxyprop-1-(Z)-enyl-17-β-hydroxy-Eduktes.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem. 18 (1978) 259-260.

Auch die Einführung der 17-Hydroxyacetylseitenkette erfolgt nach an sich bekannten Methoden, beispielsweise nach den in J. Org. Chem. 47 (1982), 2993-2995, Chem.Ber. 113 (1984), 1184 bzw. US-Patent 4.600.538 beschriebenen Methoden.

Zur Einführung der Gruppierungen

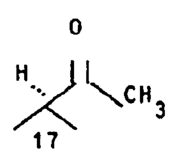

und

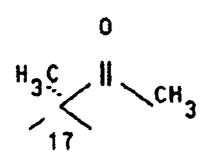

wird das 17-Keton mit Tosylmethylisocyanid (Chem. Ind. 1972 213) in die 17-Nitrilverbindung (Tetrahedron 31(1975),2151) überführt, das direkt mit Methyllithium oder Methylmagnesiumbromid in die 17-Acetylverbindung umgewandelt werden kann, welche nach Enolisierung mit K-tert.-Butylat in Tetrahydrofuran und Umsetzung mit Methyljodid die gewünschte 17α-Methyl-17β-acylgruppierung liefert. Diese Sequenz Methyladdition an das Nitril und anschließende Alkylierung kann auch in umgekehrter Folge ausgeführt werden.

Freie Hydroxygruppen in 17-Stellung und in den für $R^1$, $R^5$, $R^6$ und Y stehenden Resten können in an sich bekannter Weise verestert oder verethert werden.

Die Herstellung von Edukten der allgemeinen Formel II soll detailliert an 7 Verbindungen beispielhaft beschrieben werden :

**Beispiel 1 :**

17-(Prop-1-inyl)-17β-hydroxy-11β-ethinyl-4-estren-3-on

a) 17-(Prop-1-inyl)-11β-ethinyl-3-ethoxy-3,5-estradien-17β-ol : 1,2 l abs. Tetrahydrofuran (THF) werden bei einer Temperatur von 0°C mit Propin gesättigt, und zu dieser Lösung werden dann 153 ml (245 mmol) einer 1,6 m n-Butyllithiumlösung (Hexan) langsam zugegeben. Nach dreißigminütigem Nachrühren wird zu dieser Lösung eine Lösung von 7,91 g (24,4 mmol) 11β-Ethinyl-3-ethoxy-3,5-estradien-17-on (dargestellt nach DE 28 05 490 A 1) in 240 ml abs. THF zugetropft. Nach zweistündiger Reaktionszeit wird das Reaktionsgemisch auf Wasser gegossen und die wässrige Phase mit Essigester extrahiert. Die vereinigten organischen Phasen werden mit gesättigter Natriumchloridlösung gewaschen, über Natriumsulfat getrocknet und am Wasserstrahlvakuum eingeengt. Es werden 8,8 g Rohprodukt erhalten.

b) 10 g des unter a) dargestellten Rohproduktes (27,43 mmol) werden in 1 l Aceton gelöst und mit 50 ml 4n Salzsäure versetzt. Nach einstündigem Nachrühren bei Raumtemperatur wird das Reaktionsgemisch mit 350 ml ges. Natriumhydrogencarbonatlösung versetzt und die wässrige Phase mehrmals mit Methylenchlorid extrahiert. Die vereinigten organischen Phasen werden über Natriumsulfat getrocknet und am Wasserstrahlvakuum eingeengt. Der Rückstand wird an Kieselgel mit einem Gemisch aus Hexan/Essigester chromatographiert. Es werden 7,48 g (81%) 17-(Prop-1-inyl)-17β-hydroxy-11β-ethinyl-4-estren-3-on isoliert.

Fp. 183-194°C (kristallisiert aus Diisopropylether/Methylenchlorid)

**Beispiel 2 :**

17-(Prop-1-inyl)-17β-hydroxy-11β-ethinyl-4,6-estradien-3-on

Eine Suspension von 8 g der nach Beispiel 1a) dargestellten Propinylverbindung (Rohprodukt = 21,9 mmol) in 100 ml 80% igem wässri-gen Dioxan wird mit 48 ml 10% iger Natriumcetatlösung versetzt und auf 0°C abgekühlt. Dazu werden portionsweise 3,1 g 1,3-Dibrom-5,5-dimethylhydantoin derart zugegeben, daß die Innentemperatur +3° C nicht übersteigt. Anschließend wird 30 Minuten bei 0°C nachgerührt und dann das Reaktionsgemisch auf 100 ml ges. Natriumsulfit-lösung gegossen. Die wässrige Phase wird mehrmals mit Methylenchlorid extrahiert, die vereinigten organischen Phasen werden mit ges. Natriumchloridlösung gewaschen und über Natriumsulfat getrocknet. Nach Abzug der Lösungsmittel wird das Rohprodukt in 100 ml Dimethylformamid aufgenommen und mit 4,75 g Lithiumbromid und 3,8 g Lithiumcarbonat versetzt. Das Reaktionsgemisch wird für 45 min auf 100°C erhitzt und nach dem Abkühlen auf Raumtemperatur auf 1,3 l Wasser gegossen. Die wässrige Phase wird mit 2n Salzsäure auf einen pH-Wert von 7 gebracht und 30 min auf 0°C gekühlt. Anschließend wird das Steroid abfiltriert, mit Wasser gewaschen und im Vakuum getrocknet. Dieses Rohprodukt (7,95 g = 96%) besitzt genügend Reinheit, um in den Kupplungsreaktionen eingesetzt werden zu kön-nen. Wird das Rohprodukt weiter durch eine Chromatographie an Kieselgel mit einem Gemisch aus Hexan/Essigester aufgereinigt, so isoliert man 5,73 g (78%) der gewünschten Verbindung.

Fp. : 195-198°C (kristallisiert aus Diisopropylether)

**Beispiel 3 :**

17-(Prop-1-inyl)-17β-hydroxy-11β-ethinyl-6-chlor-4,6-estradien-3-on

a) 17-(Prop-1-inyl)-17β-hydroxy-11β-ethinyl-6α,7α-epoxy-4-estren-3-on

1,04 g (3 mmol) 17-(Prop-1-inyl)-17β-hydroxy-11β-ethinyl-4,6-estradien-3-on (Beispiel 2) werden in 30 ml Methylenchlorid gelöst und bei Raumtemperatur mit 1,2 g Meta-Chlorperbenzoesäure (4,5 mmol) (67,7% ig) versetzt. Das Reaktionsgemisch wird über Nacht nachgerührt und anschließend auf Wasser gegossen. Nach Extraktion der wässrigen Phase mit Methylenchlorid werden die vereinigten organischen Phasen nacheinander mit ges. Natriumhydrogencarbonatlösung, ges. Natriumthiosulfatlösung und Wasser gewaschen. Es werden 1099 mg des gewünschten Produkts als Rohprodukt isoliert.

b) 17-(Prop-1-inyl)-17β-hydroxy-11β-ethinyl-6β-chlor-7α-hydroxy-4-estren-3-on

1 g der unter a) erhaltenen Verbindung wird bei Raumtemperatur in 26 ml Eisessig vorgelegt und mit 2 g Lithiumchlorid versetzt. Nach einer Nachrührzeit von 30 min wird das Reaktionsgemisch auf Eiswasser gegossen und die wässrige Phase mit Methylenchlorid extrahiert. Nach Waschen der vereinigten organischen Phasen mit Wasser, Trocknen über Natriumsulfat und Einengen zur Trockne werden 1,05 g Rohprodukt isoliert.

c)17-(prop-1-inyl)-17β-hydroxy-11β-ethinyl-6β-chlor-7α-mesyloxy-4-estren-3-on

1 g des unter b) erhaltenen Rohprodukts wird in 60 ml Methylenchlorid gelöst und nacheinander mit 3,2 ml (40 mmol) Pyridin und 0,7 ml (9 mmol) Methansulfonsäurechlorid bei Eisbadtemperatur versetzt. Über Nacht wird das Reaktionsgemisch unter Rühren langsam auf Raumtemperatur gebracht, anschließend auf Eiswasser gegossen und die wässrige Phase mit Methylenchlorid extrahiert. Nach Waschen der vereinigten organischen Phasen mit ges. Natriumhydrogencarbonatlösung und Trocknen über Natriumsulfat werden sie zur Trockne eingeengt und so 1560 mg Rohprodukt erhalten.

d) 17-(Prop-1-inyl)-17β-hydroxy-11β-ethinyl-6-chlor-4,6-estradien-3-on

Das unter c) erhaltene Rohprodukt wird in 60 ml abs. Dimethylformamid gelöst und mit 3,65 g Natriumacetat versetzt. Anschließend wird das Reaktionsgemisch 4 Stunden auf 100°C erhitzt, bevor es auf Wasser gegossen wird. Die wässrige Phase wird mit Essigester extrahiert. Nach Waschen der vereinigten organischen Phasen mit ges. Natriumchloridlösung und Trocknen über Natriumsulfat werden die organischen Lösungsmittel im Vakuum abgezogen und der Rückstand an Kieselgel mit einem Gemisch aus Hexan/Essigester chromatographiert. Es werden 205 mg des gewünschten Produkts erhalten.

H-NMR($CDCl_3$) : δ 6,3-6,45(2H, m, H – 4 + H – 7) ; 3,0-3,15(1H, m, H – 11) ;
1,85(3H, s, $CH_3$ – CℓC) ; 1,22(3H, s, H – 18).

**Beispiel 4 :**

17-Methoxymethyl-17β-hydroxy-11β-ethinyl-4-estren-3-on

a) 11β-Ethinyl-3-ethoxy-3,5-estradien-[17-(β-1′)-spiro-3′]oxiran

1.62 g (5 mmol) 11β-Ethinyl-3-ethoxy-3,5-estradien-17-on werden in 50 ml abs. Dimethylformamid gelöst, auf 0°C gekühlt und nacheinander mit 5,1 g (25 mmol) Trimethylsulfoniumjodid und 2,9 g (26 mmol) Kaliumtert.butylat versetzt. Das Reaktionsge-misch wird über Nacht nachgerührt und dabei langsam auf Raumtemperatur erwärmt. Anschließend wird es auf Wasser gegossen und die wässrige Phase mehrmals mit Essigester extrahiert. Die ver-einigten organischen Phasen werden mit ges. Natriumchloridlösung gewaschen, über Natriumsulfat getrocknet und im Vakuum zur Trockne eingeengt. Es werden 1,44 g Rohprodukt isoliert.

b) 17-Methoxymethyl-11β-ethinyl-3-ethoxy-3,5-estradien-17β-ol

1,35 g (4 mmol) des unter a) erhaltenen Rohprodukts werden gelöst in 20 ml Methanol und zu 40 ml einer 3 m Natriummethyllatlösung (methanolisch) zugegeben. Das Reaktionsgemisch wird anschließend solange unter Rückfluß er hitzt, bis das Startmaterial vollständig umgesetzt ist. Danach wird es auf Wasser gegossen, die wässrige Phase mit Essigester extrahiert und die organische Phase mit Natriumchloridlösung gewaschen. Nach Trocknen über Natriumsulfat und Abzug der Lösungsmittel in Vakuum werden 1,45 g des gewünschten Produkts als Rohprodukt erhalten.

c) 17-Methoxymethyl-17β-hydroxy-11β-ethinyl-4-estren-3-on

1,35 g des unter b) erhaltenen Rohprodukts werden bei Raumtemperatur in 70 ml Aceton gelöst und mit 3,4 ml 4 n Salzsäure (wässrig) versetzt. Nach vollständiger Spaltung des Dienolethers (DC-Kontrolle) wird das Reaktionsgemisch auf ges. Natriumhydro-gencarbonatlösung gegossen und die wässrige Phase mit Methylen-chlorid extrahiert. Nach Trocknen der vereinigten organischen Phasen über Natriumsulfat und Entfernung des Lösungsmittel im Vakuum wird der Rückstand an Kieselgel miteinem Gemisch aus Hexan/Essigester chromatographiert. Es werden 536 mg 17-Methoxymethyl-17β-hydroxy-11β-ethinyl-4-estren-3-on isoliert.

H-NMR($CDCl_3$): δ 5,87(1H,s,H-4); 3,48 und 3,16 (jeweils 1H, d J=10Hz, O-$CH_2$); 3,36(3H,s,$OCH_3$); 3,0(1H,m,H-11); 1,23(3H,s,H-18);
$[\alpha]^{20°C}_D$= 92°($CHCl_3$)

**Beispiel 5 :**

17-(3-Methoxymethoxy-prop-1[Z]-enyl)-17β-hydroxy-11β-ethinyl-4-estren-3-on

a) 3-Ethoxy-3,5-estradien-11,17-dion

49,1 g (172 mmol) 4-Estren-3,11,17-trion (European patent application 0 145 493) werden bei 0°C in 180 ml abs. Ethanol und 300 ml Methylenchlorid gelöst und nacheinander mit 51 ml Triethylorthoformiat und 600 mg p-Toluolsulfonsäure versetzt. Das Reaktionsgemisch wird bei derselben Temperatur 5 Stunden nachgerührt und dann mit 75 ml Pyridin und 125 ml Wasser versetzt. Nach einstündigem Nachrühren bei 0°C wird die organische Phase abgetrennt und die wässrige mit Methylenchlorid extra-hiert. Die vereinigten organischen Phasen werden mit Wasser ge-waschen über Natriumsulfat getrocknet und am Vakuum eingeengt. Der Rückstand wird aus Ethanol umkristallisiert. Es werden 41 g des gewünschten Enolethers erhalten.

b) 17-(3-Hydroxyprop-1-inyl)-17β-hydroxy-3-ethoxy-3,5-estradien-11-on

30 g des unter a) dargestellten Produkts werden bei 0°C in 1,2 l abs. Tetrahydrofuran gelöst und unter Schutzgas mit 220 g Kaliumethylat versetzt. Zu dieser Suspension werden 77,4 ml Propargylalkohol zugetropft und anschließend das Reaktionsgemisch über Nacht bei 0°C nachgerührt. Zur Aufarbeitung wird es auf Wasser gegossen und die wässrige Phase mit Methylenchlorid extrahiert. Die vereinigten organischen Phasen werden mit ges. Natriumchloridlösung gewaschen, über Natriumsulfat getrocknet und am Vakuum zur Trockne eingeengt. Es werden 38,2 g Rohprodukt erhalten.

c) 17-(3-Hydroxy-prop-1-[Z]-enyl)-17β-hydroxy-3-ethoxy-3,5-estradien-11-on

32 g des unter b) erhaltenen Rohprodukts werden in einer Mischung aus 325 ml Ethanol und 32,5 ml Pyridin bei Normaldruck mit 3,2 g Palladium/Bariumsulfat (10% ig) hydriert. Nach Aufnahme eines Äquivalents Wasserstoff wird die Hydrierung durch Filtrieren über Celite abgebrochen und das Filtrat am Vakuum eingeengt. Es werden 32,5 g Rohprodukt erhalten.

d) 17-(3-Methoxymethoxyprop-1-[Z]-enyl)-17β-hydroxy-3-ethoxy-3,5-estradien-11-on

30 g des unter c) erhaltenen Produkts werden in 200 ml abs. Methylenchlorid gelöst und bei 0°C nacheinander mit 25 ml Diisopropylamin und 9,7 ml Brommethylmethylether versetzt. Anschliessend wird bis zur vollständigen Umsetzung (DC-Kontrolle) nachgerührt. Das Reaktionsgemisch wird dann auf Wasser gegossen, die wässrige Phase mit Methylenchlorid extrahiert und die vereinigten organischen Phasen werden mit ges. Natriumchloridlösung gewaschen. Nach Trocknen über Natriumsulfat werden die Lösungsmittel am Vakuum abgezogen. Der Rückstand wird an Aluminiumoxid (neutral, Stufe III) mit einem Gemisch aus Essigester/Hexan chromatographiert. Es werden 22,4 g des gewünschten Produkts isoliert.

e) 17-(3-Methoxymethoxyprop-1-[Z]-enyl)-17β-hydroxy-11-(E)-methoxymethylen-3-ethoxy-3,5-estradien

20 g des unter d) erhaltenen Produkts werden in 200 ml abs. Toluol unter Schutzgas gelöst und zum Phosphorylid, generiert durch Zugabe von 43 g Kaliumtert.butylat zu einer Suspension von 81 g Methoxymethyltriphenylphosphoniumchlorid in 600 ml abs. To- luol bei 0°C unter Schutgas, bei 5°C langsam zugetropft. Es wird solange nachgerührt, bis eine vollständige Umsetzung (DC-Kontrolle) stattgefunden hat. Anschließend wird das Reaktionsgemisch auf Wasser gegossen, die wässrige Phase mit Methylenchlorid extrahiert und die gesamte organische Phase über Natriumsulfat getrocknet. Nach Abzug der Lösungsmittel wird der Rückstand an Aluminiumoxid (neutral, Stufe III) mit einem Gemisch aus Hexan/Essigester chromatographiert. Es werden 8,4 g des gewünschten Produkts erhalten.

f) 17-(3-Methoxymethoxyprop-1-[Z]-enyl)-17β-hydroxy-11β-formyl-4-estren-3-on

8 g des unter e) dargestellten Produkts werden in 400 ml Aceton gelöst und unter Schutzgas mit 15 ml 4 n wässriger Salzsäure versetzt. Nach zweistündigem Nachrühren bei Raumtemperatur wird das Reaktionsgemisch auf ges. Natriumhydrogencarbonatlösung gegossen und die wässrige Phase mit Methylenchlorid extrahiert. Die vereinigten organischen Phasen werden mit Wasser gewaschen und über Natriumsulfat getrocknet. Nach Abzug der Lösungsmittel im Vakuum werden 6,8 g Rohprodukt erhalten.

g) 17-(3-Methoxymethoxyprop1-[Z]-enyl)-17β-hydroxy-11β-(2-brom-[Z]-vinyl)-4-estren-3-on

Unter Schutzgas werden 34,2 g Brommethyltriphenylphosphoniumbromid, suspendiert in 500 ml abs. Tetrahydrofuran, mit 8,8 g Kaliumtert.butylat bei −60°C versetzt und 15 min nachgerührt. 6,5 g des unter f) erhaltenen Rohprodukts werden in 75 ml abs. Tetrahydrofuran gelöst und bei −60°C zum gebildeten Ylid langsam zugetropft. Nach vollständiger Umsetzung (DC-Kontrolle) wird das Reaktionsgemisch auf Wasser gegossen, die wässrige Phase mit Essigester extrahiert und die gesamte organische Phase mit ges. Natriumchloridlösung gewaschen. Nach Trocknen über Natriumsulfat werden die Lösungsmittel im Vakuum abgezogen. Der Rückstand wird anschließend an Kieselgel mit einem Gemisch aus Hexan/Essigester chromatographiert. Es werden 3,17 g des gewünschten Produkts erhalten.

h) 17-(3-Methoxymethoxyprop-1-[Z]-enyl)-17β-hydroxy-11β-ethinyl-4-estren-3-on

Unter Schutzgas werden 8,8 ml Diisopropylamin in 175 ml abs. Tetrahydrofuran vorgelegt, auf −10°C gekühlt und mit 4 ml 1,6 m n-Butyllithiumlösung (in Hexan) versetzt. Nach 15-minütigem Nachrühren bei 0°C wird zu dieser Lösung das unter g) erhaltene Steroid, gelöst in 175 ml abs. Tetrahydrofuran bei −78°C zugetropft. Nach 30-minütigem Nachrühren wird die Lösung auf Wasser gegossen und die wässrige Phase mit Methylenchlorid extrahiert. Die vereinigten organischen Phasen werden über Natriumsulfat getrocknet und im Vakuum eingeengt. Der Rückstand wird an Kieselgel mit einem Gemisch aus Hexan/Essigester chromatographiert. Es werden 2,16 g der gewünschten Verbindung erhalten.

H-NMR($CDCl_3$) : δ 5,86 (1H, s breit, H − 4) ; 5,55-5,7 (2H, m, olefin.H) ; 4,68(2H, s, O-$CH_2$-O) ; 4,2-4,45 (2H, m, O-$CH_2$-C=) ; 3,39 (3H, s, $OCH_3$) ; 1,27 (3H, s, H − 18) ;

**Beispiel 6 :**

17-(3-Hydroxyprop-1-[Z]-enyl)-17β-hydroxy-11β-ethinyl-4-estren-3-on

8 g des in Beispiel 5 erhaltenen Produkts werden in 50 ml Tetrahydrofuran gelöst und mit 30 ml 4 n Salzsäure versetzt. Das Reaktiongemisch wird über Nacht bei Raumtemperatur nachgerührt und anschließend auf ges. Natriumchloridlösung gegossen. Die wässrige Phase wird mehrmals mit Methylenchlorid extrahiert. Die vereinigten organischen Phasen werden mit ges. Natriumhydrogencarbonatlösung gewaschen, über Natriumsulfat getrocknet und im Vakuum eingeengt. Der Rückstand wird an Kieselgel mit einem Gemisch aus Hexan/Essigester chromatographiert. Es werden 5,62 des gewünschten Produkts erhalten.

H-NMR($CDCl_3$) : δ 5,88(1H, s breit, H − 4) ; 5,55-5,78(2H, m,H-olefin.) ; 4,29(2H, m, O-$CH_2$-C=) ; 1,26(3H, s, H − 18) ;

**Beispiel 7 :**

11β-Ethinyl-4-estren [17(β-1′)-spiro-5′]-2′,5′-dihydrofuran-3-on

5,3 g 17-(3-Hydroxyprop-1-[Z]-enyl)-17β-hydroxy-11β-ethinyl-4-estren-3-on (Beispiel 6) werden in 75 ml abs. Methylenchlorid vorgelegt und mit 14 ml Triethylamin versetzt. Die Lösung wird anschließend auf 0°C gekühlt und mit 2 ml Methansulfonsäurechlorid durch langsames Zutropfen versetzt. Nach Beendigung der Zugabe wird das Reaktionsgemisch 1 Stunde nachgerührt und dann auf ges. Natriumhydrogencarbonatlösung gegossen. Die wässrige Phase wird mehrmals mit Methylenchlorid extrahiert. Die vereinigten organischen Phasen werden mit ges. Natriumchloridlösung gewaschen, über Natriumsulfat getrocknet und im Vakuum eingeengt. Der Rückstand wird an Kieselgel mit einem Gemisch aus Hexan/Essigester chromatographiert. Es werden 4,05 g des gewünschten Produkts isoliert.

H-NMR($CDCl_3$) : δ 5,7-5,95 (3H, m, H − 4 + olefin. Protonen) ; 4,5-4,62(2H, m, O-$CH_2$C=) ; 1,26(3H, s, H − 18)

Fp. : 139-144°C (Essigester, Diisopropylether)

Die neuen Verbindungen der allgemeinen Formel I sind wertvolle Pharmaka. So verfügen sie über eine starke Affinität zum Gestagenrezeptor und besitzen einen überraschend großen Bereich an gestagenen, antigestagenen, antiglucocorticoiden und antimineralcorticoiden Eigenschaften. Diese wichtigen biologischen Wirksamkeiten können für medizinische Zwecke genutzt werden.

Wirkstoffe dieser Art mit ausgeprägter antigestagener Aktivität sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblock auf ihre Verwendung zur postcoitalen Fertilitätskontrolle. Sie können auch gegen hormonelle Unregelmäßigkeiten, zur Menstruationsauslösung und zur Geburtseinleitung eingesetzt werden. Außerdem können sie für die Behandling von hormonabhängigen Carcinomen eingesetzt werden.

Die erfindungsgemäßen Verbindungen der allgemeinen Formel I weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom) eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Die erfindungsgemäßen Verbindungen der allgemeinen Formel I mit gestagener Aktivität können beispielsweise bei der Therapie von Amenorrhoe, Dysmenorrhoe, Hypermenorrhoe und lutealer Insuffizienz, solche mit antimineralcorticoiden Eigenschaften zur Behandlung von Krankheitszuständen, an denen ein Hyperaldosteronismus beteiligt ist, verwendet werden.

Die Erfindung betrifft somit auch Arzneimittel auf Basis der pharmazeutisch verträglichen, d.h. in den verwendeten Dosen nicht toxischen Verbindungen der allgemeinen Formel I, gegebenenfalls zusammen mit den üblichen Hilfs- und Trägerstoffen.

Die erfindungsgemäßen Verbindungen können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben Cremes und Gelen verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens® oder Myrj®, Magnesiumstearat, wäßrigen oder nichtwäßrigen Trägern, Paraffinderivaten, Netz-, Dispergier-, Emulgier-, Konservie-rungsmitteln und Aromastoffen zur Geschmackskorrektur (z.B. ätherischen Ölen) gemischt werden.

Die Erfindung betrifft somit auch pharmazeutische Zusammensetzungen, die als Wirkstoff zumindest eine erfindungsgemäße Verbindung enthalten.

Eine Dosiseinheit enthält etwa 0,01-1000 mg Wirkstoff(e).

Die Dosierung der erfindungsgemäßen Verbindungen liegt beim Menschen bei etwa 0,04-10000 mg pro Tag.

Allgemeine Vorschrift zur Palladium-katalysierten Kupplung

Unter Schutzgas werden y mmol der Acetylenkomponente in y x 45 ml Triethylamin gelöst und mit yx 10 mmol des jeweiligen Kupplungspartners versetzt. Nach Zugabe von 10 mol% Palladiumtetrakistriphenylphosphin und 5 mol% Kupfer (I) jodid wird solange auf 60% C erhitzt, bis eine vollständige Umsetzung (DC-Kontrolle) erzielt worden ist. Anschließend wird das Reaktionsgemisch zur Entfernung des Katalysators über Celite filtriert, das Filtrat zur Trockne eingeengt und der Rückstand an Kieselgel mit Hexan/Essigester chromatographiert.

| Beispiel | Y [mMol] | Ausbeute % | $[\alpha]_D^{20°}$ ($C=0,5;CHCl_3$) | $R^{1'}V$ |
|---|---|---|---|---|
| 17-(Prop-1-inyl)-17β-hydroxy-11β-[2-(phenyl)-ethinyl]-4-estren-3-on | 2 | 82 | +118° | Jodbenzol |
| 17-(Prop-1-inyl)-17β-hydroxy-11β-[2-(4-dimethyl-aminophenyl)-ethinyl]-4-estren-3-on | 2 | 31 | +146° | 4-Brom-N-N-dimethylanilin |
| 17-(Prop-1-inyl)-17β-hydroxy-11β-[-2-(4-methoxy-phenyl)-ethinyl]-4-estren-3-on | 2 | 75 | +135° | 4-Jodanisol |
| 17-(Prop-1-inyl)-17β-hydroxy-11β-[2-(4-cyanophenyl)-ethinyl]-4-estren-3-on | 2 | 76 | +130° | 4-Brombenzonitril |
| 17-(Prop-1-inyl)-17β-hydroxy-11β-[2-(4-acetylphenyl)-ethinyl]-4-estren-3-on | 2 | 90 | +147° | 4-Bromacetophenon |
| 17-(Prop-1-inyl)-17β-hydroxy-11β-[2-(4-acetylphenyl)-ethinyl]-4,6-estradien-3-on | 2 | 18 | +300° | 4-Bromacetophenon |
| 17-(Prop-1-inyl)-17β-hydroxy-11β-[2-(4-methoxyphenyl)-ethinyl]-4,6-estradien-3-on | 2 | 55 | +239° | 4-Jodanisol |

| Beispiel | Y [mMol]] | Ausbeute % | $[\alpha]_D^{20°}$ (C=0,5; $CHCl_3$) | R[1]'V |
|---|---|---|---|---|
| 17-(Prop-1-inyl)-17β-hydroxy-11β-[2-(2-thienyl)-ethinyl]-4-estren-3-on | 2 | 44 | +145° | 2-Bromthiophen |
| 17-(Prop-1-inyl)-17β-hydroxy-11β-[2-(3-thienyl)-ethinyl]-4-estren-3-on | 2 | 43 | +127° | 3-Bromthiophen |
| 17-(Prop-1-inyl)-17β-hydroxy-11β-[2-(5-formyl-2-thienyl]-ethinyl]-4-estren-3-on | 2 | 92 | +155° | 2-Brom-5-formyl-thiophen |
| 17-(Prop-1-inyl)-17β-hydroxy-11β-[-2-(3-pyridinyl)-ethinyl]-4-estren-3-on | 2 | 81 | +133° | 3-Jodpyridin |
| 17-(Prop-1-inyl)-17β-hydroxy-11β-[2-(4-pyridinyl)-ethinyl]-4-estren-3-on | 2 | 20 | +124° | 4-Brompyridin-hydrochlorid |
| 17-(Prop-1-inyl)-17β-hydroxy-11β-[2-(5-acetyl-2-thienyl)-ethinyl]-4-estren-3-on | 2 | 86 | +167° | 2-Brom-5-acetyl-thiophen |
| 17-(Prop-1-inyl)-17β-hydroxy-11β-(2-phenylethinyl)-4,6-estradien-3-on | 2 | 68 | +235° | Jodbenzol |

| Beispiel | Y [mMol] | Ausbeute % | $[\alpha]_D^{20°}$ (C=0,5;$CHCl_3$) | $R^{1'}V$ |
|---|---|---|---|---|
| 17-(Prop-1-inyl)-17β-hydroxy-11β-[2-(5-isopropyloxycarbonyl-2-furyl)-ethinyl]-4,6-estradien-3-on | 2 | 66 | +234° | 2-Bromfuran-5-carbonsäureiso-propylester |
| 17-(Prop-1-inyl)-17β-hydroxy-11β-[2-(3-nitrophenyl)-ethinyl]-4-estren-3-on | 2 | 78 | +118° | 3-Bromnitroben-zol |
| 17-(Prop-1-inyl)-17β-hydroxy-11β-[2-(2-nitrophenyl)-ethinyl]-4-estren-3-on | 2 | 71 | +108° | 2-Bromnitroben-zol |
| 17-(Prop-1-inyl)-17β-hydroxy-11β-[2-(2-methoxyphenyl)-ethinyl]-4-estren-3-on | 2 | 94 | +145 | 2-Jodanisol |
| 17-(Prop-1-inyl)-17β-hydroxy-11β-[2-(p-tolyl)-ethinyl]-4-estren-3-on | 2 | 83 | +128° | 4-Jodtoluol |
| 17-(Prop-1-inyl)-17β-hydroxy-11β-[2-(o-tolyl)-ethinyl]-4-estren-3-on | 2 | 95 | +131 | 2-Jodtoluol |

| Beispiel | Y [mMol] | Ausbeute % | $[\alpha]_D^{20°}$ ($C=0,5;CHCl_3$) | $R^{1'}V$ |
|---|---|---|---|---|
| 17-(Prop-1-inyl)-17β-hydroxy-11β-[2-(5-isopropoxy-carbonyl-2-furyl)-ethinyl]-4-estren-3-on | 2 | 65 | +122 | 2-Bromfuran-5-carbonsäureiso-propylester |
| 17-(Prop-1-inyl)-17β-hydroxy-11β-[2-(4-fluorphenyl)-ethinyl]-4,6-estradien-3-on | 2 | 58 | +204 | 4-Fluorjodbenzol |
| 17-(Prop-1-inyl)-17β-hydroxy-11β-[2-(4-isopropylphenyl)-ethinyl]-4,6-estradien-3-on | 2 | 98 | +225 | 4-Jodisopropyl-benzol |
| 11β-[2-(4-Methoxyphenyl)-ethinyl]-4-estren-[17-(β-1')-spiro-5']-2',5'-dihydrofuran-3-on | 2 | 54 | +195 | 4-Jodanisol |
| 11β-[2-Tolyl)-ethinyl]-4-estren-[17-(β-1')-spiro-5']-2',5'-dihydrofuran-3-on | 2 | 73 | +210 | 4-Jodtoluol |
| 17-(Prop-1-inyl)-17β-hydroxy-11β-(3-methylbut-1-in-3-enyl)-4,6-estradien-3-on | 2 | 94 | +134 | 2-Brompropen |
| 17-(Prop-1-inyl)-17β-hydroxy-11β-[2-(3-dimethylaminophenyl)-ethinyl]-4-estren-3-on | 2 | 34 | +114 | 3-Brom-N,N-dimethyl-anilin |
| 17-(Prop-1-inyl)-17β-hydroxy-11β-[2-(m-tolyl)-ethinyl]-4-estren-3-on | 2 | 90 | +118 | 3-Bromtoluol |
| 17-(Prop-1-inyl)-17β-hydroxy-11β-[2-(3,5-dimethylphenyl)-ethinyl]-4-estren-3-on | 2 | 83 | +110 | 5-Jod-m-xylol |

| Beispiel | Y [mMol] | Ausbeute % | $[\alpha]_D^{20°}$ (C=0,5; $CHCl_3$) | $R^{1'}V$ |
|---|---|---|---|---|
| 17-(3-Methoxymethoxyprop-1(Z)-enyl)-17β-hydroxy-11β-[2-(2-methoxyphenyl)-ethinyl]-4-estren-3-on | 2 | 88 | +140 | 2-Jodanisol |
| 17-(3-Hydroxyprop-1(Z)-enyl)-17β-hydroxy-11β-[2-(2-methoxyphenyl)-ethinyl]-4-estren-3-on | 2 | 65 | +130 | 2-Jodanisol |
| 17-(Prop-1-inyl)-17β-hydroxy-11β-[2-(3-acetylphenyl)ethinyl]-4-estren-3-on | 2 | 95 | +121 | 3-Jodaceto-phenon |
| 17-(Prop-1-inyl)-17β-hydroxy-11β-[2-(1-naphthyl)-ethinyl]-4-estren-3-on | 2 | 91 | +148 | 1-Jodnaphtha-lin |
| 17-(Prop-1-inyl)-17β-hydroxy-11β-[2-(3-methoxyphenyl)-ethinyl]-4-estren-3-on | 2 | 93 | +132 | 3-Jodanisol |

17-(Prop-1-inyl)-17β-hydroxy-11β-[2-(phenyl)-ethinyl]-4-estren-3-on-anti-oxim
und
17-(Prop-1-inyl)-17β-hydroxy-11β-[2-(phenyl)-ethinyl]-4-estren-3-on-syn-oxim

660 mg (1,6 mmol) 17-(Prop-1-inyl)-11β[2-(phenyl)-ethinyl]-4-estren-3-on werden in 10 ml Pyridin gelöst und bei 0°C portionsweise mit 560 mg Hydroxylaminhydrochlorid versetzt. Nach Zugabe rührt man 30 Minuten bei +5°C, gießt in eine Mischung aus Eiswasser/0,5 n-Salzsäure und extrahiert mit dichlormethan. Die vereinigten organischen Phasen werden im Vakuum eingeengt. Das Rohprodukt wird an Kieselgel mit einem Gemisch aus Hexan/Essigester chromatographiert. Es werden 347 mg 17-(Prop-1-inyl)-17β-hydroxy-11β-[2-(phenyl)-ethinyl]-4-estren-3-on-anti-oxim und 155 mg 17-(Prop-1-inyl)-17β-hydroxy-11β-[2-(phenyl)-ethinyl]-4-estren-3-on-syn-oxim isoliert.

17-(Prop-1-inyl)-17β-hydroxy-11β-[2-(phenyl)-ethinyl]-4-estren-3-on-anti-oxim

H-NMR($CDCl_3$) : δ 7,3-7,5 (5H, m, aromat. Protonen) ; 5,9 (1H,s breit, H – 4) ; 3,3-(1H, m, H – 11) ; 1,85 (3H, s, $CH_3$-C≡C) ; 1,25(3H, s, H – 18) ;

17-(Prop-1-inyl)-17β-hydroxy-11β-[2-(phenyl)-ethinyl]-4-estren-3-on-syn-oxim

H-NMR($CDCl_3$) : δ 7,3-7,5 (5H,m, aromat. Protonen) ; 5,55 (1H,s breit,H-4) ; 3,25 (1H,m,H-11) ; 1,85 (3H,s,$CH_3$-C≡C) ; 1,25 (3H,s,H-18).

## Ansprüche

1. 11β-substituierte Steroide der allgemeinen Formel I

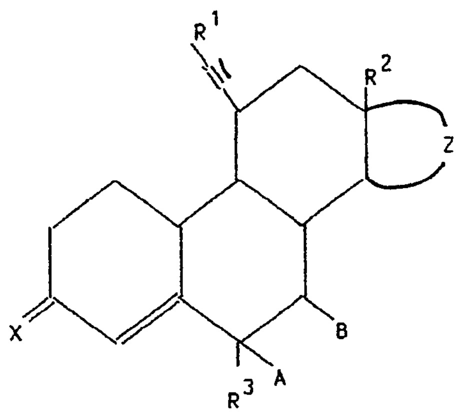

(I),

worin

A und B gemeinsam für eine zweite Bindung zwischen den Kohlenstoffatomen 6 und 7 oder jeweils für ein Wasserstoffatom,

X für ein Sauerstoffatom, zwei Wasserstoffatome oder die Hydroxyiminogruppierung N~OH,

Z für den Rest eines Rings der Formel

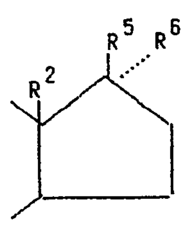

,

worin

$R^5/R^6$

$-OR^7/-C \equiv C-Y$

$-OR^7/-\underset{\underset{O}{\|}}{C}-CH_2-R^8$

$-\underset{\underset{O}{\|}}{C}-CH_2-R^8/-OR^7$

$-\underset{\underset{O}{\|}}{C}-CH_2-R^8/-CH_3$

$-\underset{\underset{O}{\|}}{C}-CH_2-R^8/-H$

$-OR^7/-(CH_2)_m-CH_2-R^9$

$-OR^7/-CH=CH(CH_2)_k-CH_2-R^9$

$-OR^{10}/-H$

oder einen Rest der Formel

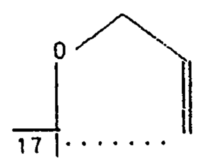

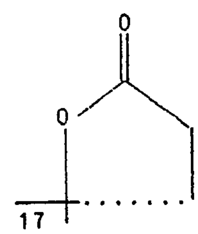

bezeichnet, mit

$R^7$ in der Bedeutung eines Wasserstoffatoms oder Arylrestes mit 1 bis 4 Kohlenstoffatomen,

Y in der Bedeutung eines Wasserstoffatoms, einer Alkyl-, Hydroxyalkyl-, Alkoxyalkyl- oder Aryloxyalkylgruppe mit jeweils 1 bis 4 Kohlenstoffatomen im Alkyl- bzw. Acylrest,

$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,

$R^9$ in der Bedeutung eines Hydroxy- oder Cyanidrestes, einer O-Alkyl- oder O-Acylgruppe mit jeweils 1 bis 4 Kohlenstoffatomen, die gegebenenfalls substituiert ist durch eine $C_1$-$C_4$-O-Alkylgruppe,

$R^{10}$ in der Bedeutung eines Wasserstoffatoms, einer Alkyl- oder Acylgruppe mit jeweils 1 bis 10 Kohlenstoffatomen,

m in der Bedeutung 0, 1, 2 oder 3,

k in der Bedeutung 0, 1, oder 2,

$R^1$ für einen Vinyl-oder Cyclo-1-alkenyl-Rest ;

eine Phenyl-, Naphthylgruppe oder einen 5-oder 6-gliedrigen Aromaten mit wenigstens einem Stickstoff-, Sauerstoff- oder Schwefelatom ; einen Vinyl-oder Cyclo-1-alkenyl-rest, eine Phenyl-, Naphthylgruppe oder einen 5- oder 6-gliedrigen Aromaten mit wenigstens einem Stickstoff-, Sauerstoff- oder Schwefelatom, substituiert durch 1-3 Halogen-atome, 1-3 $C_1$-$C_4$-Alkylgruppen, eine $C_1$-$C_4$-O-Alkyl-, $C_2$-$C_{10}$-Alkenyl-, $C_1$-$C_4$-Acyl-, $C_1$-$C_4$-O-Acyl-, eine gegebenenfalls durch eine oder zwei $C_1$-$C_4$-Alkylgruppeln) substituierte Aminogruppe, einen Phenyl-, Nitro-, Hydroxy-, Carboxy-, Cyanid- oder $COOR^4$-Rest mit $R^4$ in der Bedeutung einer $C_1$-$C_4$-Alkylgruppe, die gegebenenfalls substituiert ist durch eine gegebenenfalls mit einem $C_1$-$C_4$-Alkyl-, $C_1$-$C_4$-O-Alkyl-, Halogen- oder Phenyl-Rest substituierte Phenylgruppe,

$R^2$ für eine Methyl-oder Ethylgruppe,

$R^3$ für ein Wasserstoff-, Chloratom oder eine Methylgruppe stehen

2. Verbindungen gemäß Anspruch 1 dadurch gekennzeichnet, daß $R^3$, A und B jeweils für ein Wasserstoffatom stehen.

3. Verbindungen gemäß Anspruch 1, dadurch gekennzeichnet, daß A und B gemeinsam für eine zweite Bindung und $R^3$ für ein Wasserstoffatom stehen.

4. Verbindungen gemäß Anspruch 1, dadurch gekennzeichnet, daß A und B gemeinsam für eine zweite Bindung und $R^3$ für ein Chloratom stehen.

5. Verbindungen gemäß Anspruch 1, dadurch gekennzeichnet, daß A und B gemeinsam für eine zweite Bindung und $R^3$ für eine Methylgruppe stehen.

6. Verfahren zur Herstellung von Verbindungen der allgemeinen Formel I

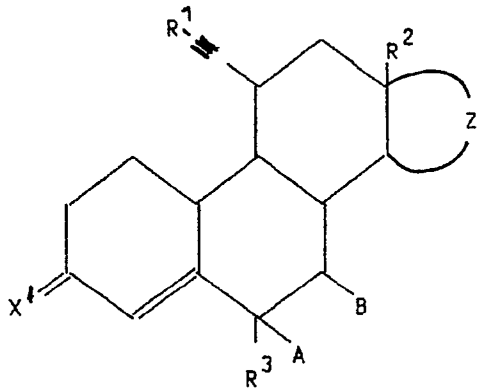

(I),

worin

A und B gemeinsam für eine zweite Bindung zwischen den Kohlenstoffatomen 6 und 7 oder jeweils für ein Wasserstoffatom,

X für ein Sauerstoffatom, zwei Wasserstoffatome oder die Hydroxyiminogruppierung N ~OH,

Z für den Rest eines Rings der Formel

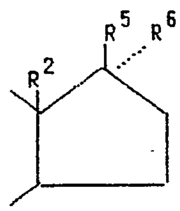

,

worin

| | |
|---|---|
| $R^5/R^6$ | $-OR^7/-C\equiv C-Y$ |
| | $-OR^7/-C(=O)-CH_2-R^8$ |
| | $-C(=O)-CH_2-R^8/-OR^7$ |
| | $-C(=O)-CH_2-R^8/-CH_3$ |
| | $-C(=O)-CH_2-R^8/-H$ |
| | $-OR^7/-(CH_2)_m-CH_2-R^9$ |
| | $-OR^7/-CH=CH(CH_2)_k-CH_2-R^9$ |
| | $-OR^{10}/-H$ |

oder einen Rest der Formel

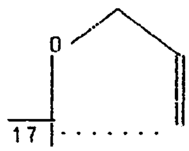

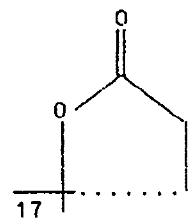

bezeichnet, mit

$R^7$ in der Bedeutung eines Wasserstoffatoms oder Acylrestes mit 1 bis 4 Kohlenstoffatomen,

Y in der Bedeutung eines Wasserstoffatoms, einer Alkyl-, Hydroxyalkyl-, Alkoxyalkyl- oder Acyloxyalkylgruppe mit jeweils 1 bis 4 Kohlenstoffatomen im Alkyl- bzw. Acylrest,

$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,

$R^9$ in der Bedeutung eines Hydroxy- oder Cyanidrestes, einer O-Alkyl- oder O-Acylgruppe mit jeweils 1 bis 4 Kohlenstoffatomen, die gegebenenfalls substituiert ist durch eine $C_1$-$C_4$-O-Alkylgruppe,

$R^{10}$ in der Bedeutung eines Wasserstoffatoms, einer Alkyl- oder Acylgruppe mit jeweils 1 bis 10 Kohlenstoffatomen,

m in der Bedeutung 0, 1, 2 oder 3,

k in der Bedeutung 0, 1, oder 2,

$R^1$ für einen Vinyl-oder Cyclo-1-alkenyl-Rest ; eine Phenyl-, Naphthylgruppe oder einen 5-oder 6-gliedrigen Aromaten mit wenigstens einem Stickstoff-, Sauerstoff- oder Schwefelatom ; einen Vinyl- oder Cyclo-1-alkenylrest, eine Phenyl-, Naphthylgruppe oder einen 5- oder 6-gliedrigen Aromaten mit wenigstens einem Stickstoff-, Sauerstoff- oder Schwefelatom, substituiert durch 1-3 Halogenatome, 1-3 $C_1$-$C_4$-Alkylgruppen, eine $C_1$-$C_4$-O-Alkyl-, $C_2$-$C_{10}$-Alkenyl-, $C_1$-$C_4$-Acyl-, $C_1$-$C_4$-O-Acyl-, eine gegebenenfalls durch eine oder zwei $C_1$-$C_4$-Alkylgruppe(n) substituierte Aminogruppe, einen Phenyl-, Nitro-, Hydroxy-, Carboxy-, Cyanid-oder $COOR^4$-Rest mit $R^4$ in der Bedeutung einer $C_1$-$C_4$-Alkylgruppe, die gegebenenfalls substituiert ist durch eine gegebenenfalls mit einem $C_1$-$C_4$-Alkyl-, $C_1$-$C_4$-O-Alkyl-, Halogen- oder Phenyl-Rest substituierte Phenylgruppe,

$R^2$ für eine Methyl- oder Ethylgruppe,

$R^3$ für ein Wasserstoff-, Chloratom oder eine Methylgruppe stehen, dadurch gekennzeichnet, daß man eine Verbindung der allgemeinen Formel II

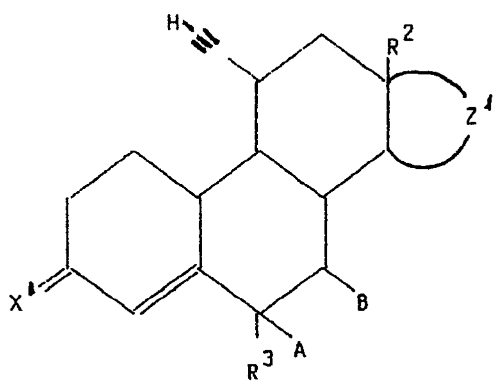

(II),

worin

A, B, $R^2$ und $R^3$ die oben genannte Bedeutung haben, X′ für ein Sauerstoffatom oder zwei Wasserstoffatome steht und Z′ die gleiche Bedeutung wie Z hat, wobei jedoch in Z gegebenenfalls vorhandene acetylenische Wasserstoffatome in Z′ geschützt sind sowie in Z gegebenenfalls vorhandene O-Acylgruppen in Z′ als OH-Gruppen vorliegen und in Z oder Z′ gegebenenfalls vorhandene OH-Gruppen gewünschtenfalls geschützt sind, mit einem Halogenid der allgemeinen Formel III

$$R^{1'}\text{-}V \quad (III)$$

worin

$R^{1'}$ die gleiche Bedeutung wie $R^1$ hat, wobei jedoch in $R^1$ gegebenenfalls vorhandene O-Acylgruppen in

$R^{1'}$ als OH-Gruppen vorliegen und in $R^1$ oder $R^{1'}$ gegebenenfalls vorhandene OH-Gruppen gewünschtenfalls geschützt sind und
V für ein Chlor-, Brom- oder Jodatom steht,
bei 0 bis 100°C in einem polaren Lösungsmittel unter Einwirkung eines Kupplungsmittels umsetzt, das so erhaltene Produkt von gegebenenfalls vorhandenen terminalen Acetylenschutzgruppen befreit, gegebenenfalls von den OH-Schutzgruppen befreit und gewünschtenfalls die in $R^1$, Y und Z vorhandenen Hydroxygruppen unter Bildung des Produkts der allgemeinen Formel I mit X in der Bedeutung von X′ verestert oder verethert und gewünschtenfalls anschließend mit Hydroxylamin-hydrochlorid in Gegenwart von tertiären Aminen bei Temperaturen zwischen −20°C und +40°C zum Produkt der allgemeinen Formel I mit X in der Bedeutung der Hydroxyiminogruppierung N~OH umsetzt.

7. Pharmazeutische Präparate, gekennzeichnet durch einen Gehalt an Verbindungen gemäß den Ansprüchen 1 bis 5.

8. Verwendung von Verbindungen gemäß den Ansprüchen 1 bis 5 zur Herstellung von Arzneimitteln

**Claims**

1. 11β-substituted steroids of the general formula I

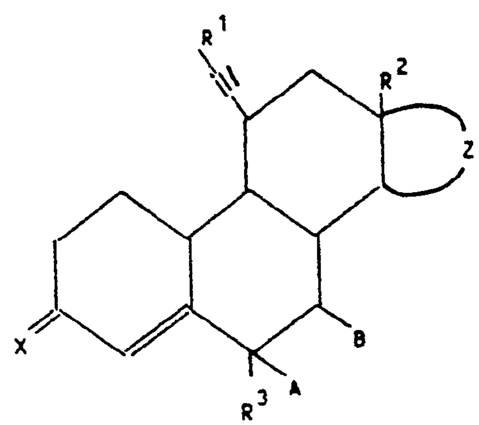

(I),

in which
A and B together represent a second bond between the carbon atoms 6 and 7 or each represents a hydrogen atom,
X represents an oxygen atom, two hydrogen atoms or the hydroxyimino grouping N~OH,
Z represents the radical of a ring of the formula

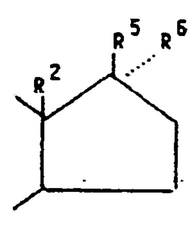

in which
$R^5/R^6$ denotes

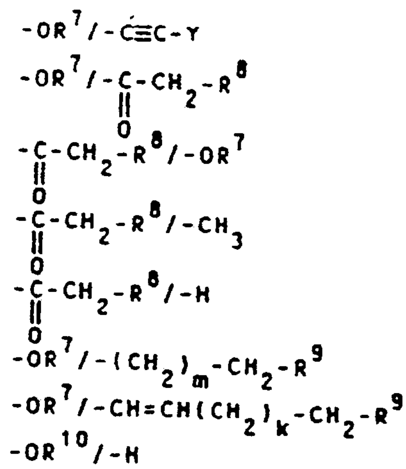

or a radical of the formula

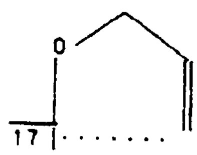

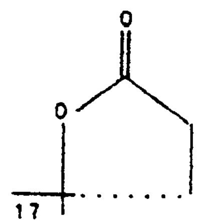

in which

$R^7$ represents a hydrogen atom or an acyl radical having from 1 to 4 carbon atoms,

Y represents a hydrogen atom, or an alkyl, hydroxyalkyl, alkoxyalkyl or acyloxyalkyl group each having from 1 to 4 carbon atoms in the alkyl or acyl radical as the case may be,

$R^8$ represents a hydrogen atom, a hydroxy group, or an alkyl, O-alkyl or O-acyl group each having from 1 to 4 carbon atoms

$R^9$ represents a hydroxy or cyanide radical, or an Oalkyl or O-acyl group each having from 1 to 4 carbon atoms, which is optionally substituted by a $C_1$-$C_4$-O-alkyl group,

$R^{10}$ represents a hydrogen atom, or an alkyl or acyl group each having from 1 to 10 carbon atoms,

m represents 0, 1, 2 or 3,

k represents 0, 1 or 2,

$R^1$ represents a vinyl or cyclo-1-alkenyl radical ; a phenyl or naphthyl group or a 5- or 6-membered aromatic group having at least one nitrogen, oxygen or sulphur atom ; a vinyl or cyclo-1-alkenyl radical, a phenyl or naphthyl group or a 5- or 6-membered aromatic group having at least one nitrogen, oxygen or sulphur atom, substituted by from 1 to 3 halogen atoms, from 1 to 3 $C_1$-$C_4$-alkyl groups, a $C_1$-$C_4$-O-alkyl group, $C_2$-$C_{10}$-alkenyl group, $C_1$-$C_4$-acyl group or $C_1$-$C_4$-O-acyl group, an amino group optionally substituted by one or two $C_1$-$C_4$-alkyl groups, or a phenyl, nitro, hydroxy, carboxy, cyanide or $COOR^4$ radical in which $R^4$ represents a $C_1$-$C_4$-alkyl group that is optionally substituted by a phenyl group that is itself optionally substituted by a $C_1$-$C_4$-alkyl, $C_1$-$C_4$-O-alkyl, halogen or phenyl radical,

$R^2$ represents a methyl or ethyl group,

$R^3$ represents a hydrogen atom, a chlorine atom or a methyl group.

2. Compounds according to claim 1, characterised in that each of $R^3$, A and B represents a hydrogen atom.

3. Compounds according to claim 1, characterised in that A and B together represent a second bond and $R^3$ represents a hydrogen atom.

4. Compounds according to claim 1, characterised in that A and B together represent a second bond and $R^3$ represents a chlorine atom.

5. Compounds according to claim 1, characterised in that A and B together represent a second bond and $R^3$ represents a methyl group.

6. Process for the preparation of compounds of the general formula I

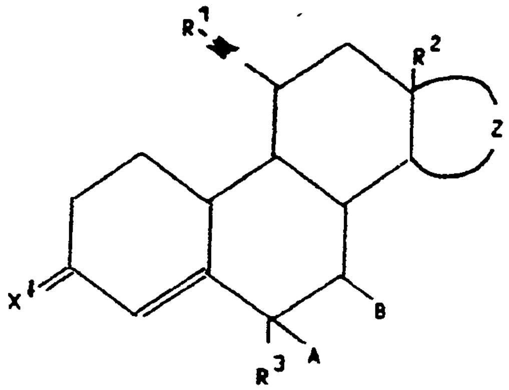

(I),

in which

A and B together represent a second bond between the carbon atoms 6 and 7 or each represents a hydrogen atom,

X represents an oxygen atom, two hydrogen atoms or the hydroxyimino grouping N ~OH,

Z represents the radical of a ring of the formula

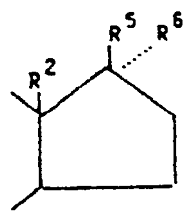

in which

$R^5/R^6$ denotes

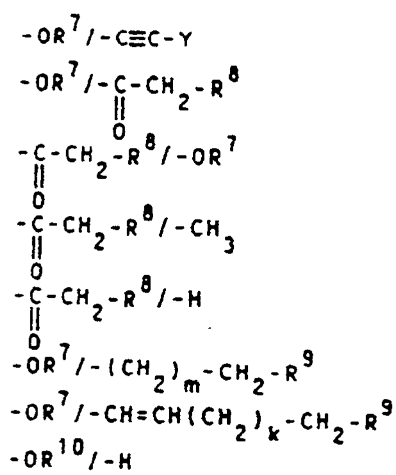

or a radical of the formula

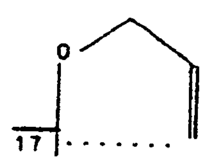

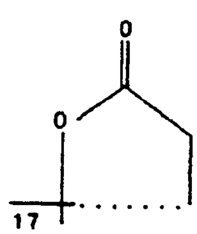

in which

$R^7$ represents a hydrogen atom or an acyl radical having from 1 to 4 carbon atoms,

Y represents a hydrogen atom, or an alkyl, hydroxy-alkyl, alkoxyalhyl or acyloxyalkyl group each having from 1 to 4 carbon atoms in the alkyl or acyl radical as the case may be,

$R^8$ represents a hydrogen atom, a hydroxy group, or an alkyl, O-alkyl or O-acyl group each having from 1 to 4 carbon atoms

$R^9$ represents a hydroxy or cyanide radical, or an O-alkyl or O-acyl group each having from 1 to 4 carbon atoms, which is optionally substituted by a $C_1$-$C_4$-O-alkyl group,

$R^{10}$ represents a hydrogen atom, or an alkyl or acyl group each having from 1 to 10 carbon atoms,

$\underline{m}$ represents 0, 1, 2 or 3,

$\underline{k}$ represents 0, 1 or 2,

$R^1$ represents a vinyl or cyclo-1-alkenyl radical ; a phenyl or naphthyl group or a 5- or 6-membered aromatic group having at least one nitrogen, oxygen or sulphur atom ; a vinyl or cyclo-1-alkenyl radical, a phenyl or naphthyl group or a 5- or 6-membered aromatic group having at least one nitrogen, oxygen or sulphur atom, substituted by from 1 to 3 halogen atoms, from 1 to 3 $C_1$-$C_4$-alkyl groups, a $C_1$-$C_4$-O-alkyl group, $C_2$-$C_{10}$-alkenyl group, $C_1$-$C_4$-acyl group or $C_1$-$C_4$-O-acyl group, an amino group optionally substituted by one or two $C_1$-$C_4$-alkyl groups, or a phenyl, nitro, hydroxy, carboxy, cyanide or $COOR^4$ radical in which $R^4$ represents a $C_1$-$C_4$-alkyl group that is optionally substituted by a phenyl group that is itself optionally substituted by a $C_1$-$C_4$-alkyl, $C_1$-$C_4$-O-alkyl, halogen or phenyl radical,

$R^2$ represents a methyl or ethyl group,

$R^3$ represents a hydrogen atom, a chlorine atom or a methyl group, characterised in that a compound of the general formula II

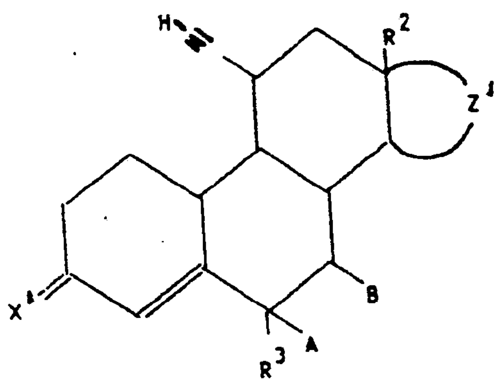

(II)

in which

A, B, $R^2$ and $R^3$ are as defined above, X′ represents an oxygen atom or two hydrogen atoms and Z′ has the same meaning as Z, except that acetylenic hydrogen atoms that may be present in Z are protected in Z′ and O-acyl groups that may be present in Z are present as OH groups in Z′, and OH groups that may be present in Z or Z′ are, if desired, protected,

is reacted with a halide of the general formula III

$$R^{1'}\text{-V} \quad \text{(III)}$$

in which

$R^{1'}$ has the same meaning as $R^1$, except that O-acyl groups that may be present in $R^1$ are present as OH groups in $R^{1'}$, and OH groups that may be present in $R^1$ or $R^{1'}$ are, if desired, protected, and

V represents a chlorine, bromine or iodine atom, at from 0 to 100°C in a polar solvent with the action of a coupling agent, the product so obtained is freed of terminal acetylene-protecting groups that may be present, if desired is freed of the OH-protecting groups and, if desired, the hydroxy groups present in $R^1$, Y and Z are esterified or etherified to form the product of the general formula I in which X has the meaning of X′ and, if desired, then reacted with hydroxylamine hydrochloride in the presence of tertiary amines at temperatures of from −20°C to +40°C to form the product of the general formula I in which X represents the hydroxyimino grouping N ~OH.

7. Pharmaceutical preparations, characterised by a content of compounds according to claims 1 to 5.

8. Use of compounds according to claims 1 to 5 for the manufacture of medicaments.

## Revendications

1. Stéroïdes substitués en position 11β de formule générale I

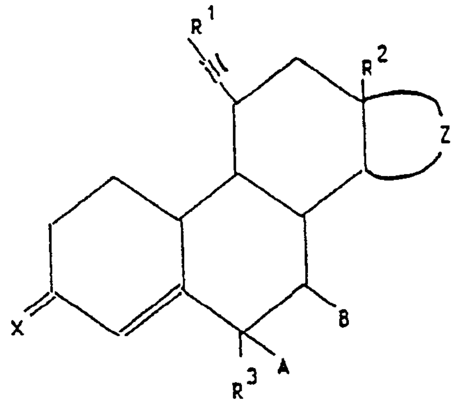

(I),

dans laquelle

A et B forment ensemble une deuxième liaison entre les atomes 6 et 7 de carbone ou bien ils représentent chacun un atome d'hydrogène,

X représente un atome d'oxygène, 2 atomes d'hydrogène ou le groupement hydroxyimino N ~OH,

Z représente le reste d'un noyau de formule

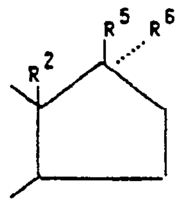

,

dans laquelle $R^5/R^6$ représentent

$-OR^7/-C{\equiv}C-Y$

$-OR^7/-\underset{\underset{O}{\|}}{C}-CH_2-R^8$

$-\underset{\underset{O}{\|}}{C}-CH_2-R^8/-OR^7$

$-\underset{\underset{O}{\|}}{C}-CH_2-R^8/-CH_3$

$-\underset{\underset{O}{\|}}{C}-CH_2-R^8/-H$

$-OR^7/-(CH_2)_m-CH_2-R^9$

$-OR^7/-CH{=}CH(CH_2)_k-CH_2-R^9$

$-OR^{10}/-H$

ou bien un reste de formule

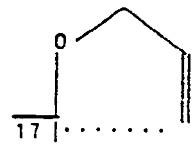

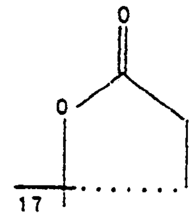

formules dans lesquelles

$R^7$ a le sens d'un atome d'hydrogène ou d'un reste acyle comportant 1 à 4 atomes de carbone, Y représente un atome d'hydrogène, un groupe alkyle, hydroxyalkyle, alcoxyalkyle ou acyloxyalkyle ayant chacun 1 à 4 atomes de carbone dans le reste alkyle ou acyle ;

$R^8$ représente un atome d'hydrogène, un groupe hydroxyle, un groupe alkyle, O-alkyle ou O-acyle ayant chacun 1 à 4 atomes de carbone,

$R^9$ représente un reste hydroxyle ou cyanure, un groupe O-alkyle ou O-acyle ayant chacun 1 à 4 atomes de carbone, pouvant éventuellement être substitué par un groupe O-alkyle en $C_1$ à $C_4$ ;

$R^{10}$ représente un atome d'hydrogène, un groupe alkyle ou acyle ayant chacun 1 à 10 atomes de carbone;

m vaut 0, 1, 2 ou 3,

k vaut 0, 1 ou 2 ;

$R^1$ représente un reste vinyle ou cycloalcényle-1, un groupe phényle, naphtyle ou un groupe aromatique à 5 ou 6 chaînons comportant au moins 1 atome d'azote, d'oxygène ou de soufre ; un reste vinyle ou cycloalcenyle-1, un groupe phényle, naphtyle ou un reste aromatique à 5 ou 6 chaînons comportant au moins un atome d'azote, d'oxygène ou de soufre, qui est substitué par 1 à 3 atomes d'halogène, 1 à 3 groupes alkyles en $C_1$ à $C_4$, un groupe O-alkyle en $C_1$ à $C_4$, alcényle en $C_2$ à $C_{10}$, acyle en $C_1$ à $C_4$, O-acyle en $C_1$ à $C_4$, un groupe amino (éventuellement substitué par un ou deux groupes alkyles en $C_1$ à $C_4$), un reste phényle, nitro, hydroxy, carboxy, cyanure ou $COOR^4$, dans lequel $R^4$ représente un groupe alkyle en $C_1$ à $C_4$, qui est éventuellement substitué par un groupe phényle (lui-même éventuellement substitué par un reste alkyle en $C_1$ à $C_4$, O-alkyle en $C_1$ à $C_4$, halogéno ou phényle) ; $R^2$ représente un groupe méthyle ou éthyle ;

$R^3$ représente un atome d'hydrogène, un atome de chlore ou un groupe méthyle.

Les groupes alkyles, alcoxy, acyles et acyloxy contenus dans les groupes représentés par $R^1$, $R^5$ et $R^6$ ou bien $R^4$, $R^7$, $R^8$, $R^9$, $R^{10}$ et Y de la formule générale I doivent contenir à chaque fois 1 à 4 atomes de carbone, et l'on préfère alors un groupe méthyle, éthyle, propyle, isopropyle, méthoxy, éthoxy, propoxy, isopropoxy, formyle, acétyle, propionyle et isopropionyle.

2. Composés selon la revendication 1, caractérisé en ce que les symboles $R^3$, A et B représentent chacun un atome d'hydrogène.

3. Composés selon la revendication 1, caractérisés en ce que A et B forment ensemble une seconde liaison, et $R^3$ représente un atome d'hydrogène.

4. Composés selon la revendication 1, caractérisés en ce que A et B forment ensemble une seconde liaison et $R^3$ représente un atome de chlore.

5. Composés selon la revendication 1, caractérisés en ce que A et B forment ensemble une seconde liaison, et $R^3$ représente un groupe méthyle.

6. Procédé pour préparer des composés de formule générale I

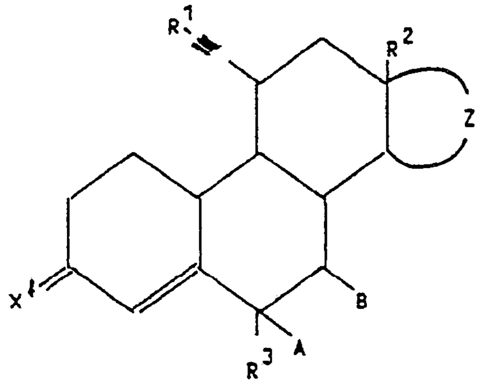

(I),

dans laquelle

A et B forment ensemble une deuxième liaison entre les atomes 6 et 7 de carbone ou bien ils représentent chacun un atome d'hydrogène,

X représente un atome d'oxygène, 2 atomes d'hydrogène ou le groupement hydroxyimino N ~OH,

Z représente le reste d'un noyau de formule

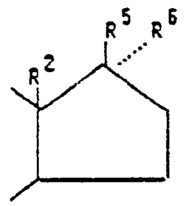

,

dans laquelle $R^5/R^6$ représentent

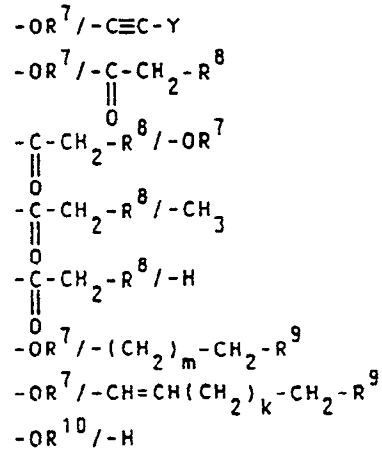

ou bien un reste de formule

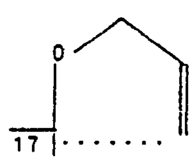

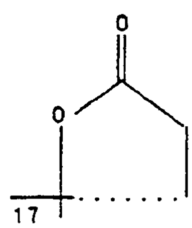

formules dans lesquelles

$R^7$ a le sens d'un atome d'hydrogène ou d'un reste acyle comportant 1 à 4 atomes de carbone, Y représente un atome d'hydrogène, un groupe alkyle, hydroxyalkyle, alcoxyalkyle ou acyloxyalkyle ayant chacun 1 à 4 atomes de carbone dans le reste alkyle ou acyle ;

$R^8$ représente un atome d'hydrogène, un groupe hydroxyle, un groupe alkyle, O-alkyle ou O-acyle ayant chacun 1 à 4 atomes de carbone,

$R^9$ représente un reste hydroxyle ou cyanure, un groupe O-alkyle ou O-acyle ayant chacun 1 à 4 atomes de carbone, pouvant éventuellement être substitué par un groupe O-alkyle en $C_1$ à $C_4$ ;

$R^{10}$ représente un atome d'hydrogène, un groupe alkyle ou acyle ayant chacun 1 à 10 atomes de carbone;

m vaut 0, 1, 2 ou 3,

k vaut 0, 1 ou 2 ;

$R^1$ représente un reste vinyle ou cycloalcényle-1, un groupe phényle, naphtyle ou un groupe aromatique à 5 ou 6 chaînons comportant au moins 1 atome d'azote, d'oxygène ou de soufre ; un reste vinyle ou cycloalcényle-1, un groupe phényle, naphtyle ou un reste aromatique à 5 ou 6 chaînons comportant au moins un atome d'azote, d'oxygène ou de soufre, qui est substitué par 1 à 3 atomes d'halogène, 1 à 3 groupes alkyles

en $C_1$ à $C_4$, un groupe O-alkyle en $C_1$ à $C_4$, alcényle en $C_2$ à $C_{10}$, acyle en $C_1$ à $C_4$, O-acyle en $C_1$ à $C_4$, un groupe amino (éventuellement substitué par un ou deux groupes alkyles en $C_1$ à $C_4$), un reste phényle, nitro, hydroxy, carboxy, cyanure ou $COOR^4$, dans lequel $R^4$ représente un groupe alkyle en $C_1$ à $C_4$, qui est éventuellement substitué par un groupe phényle (lui-même éventuellement substitué par un reste alkyle en $C_1$ à $C_4$, O-alkyle en $C_1$ à $C_4$, halogéno ou phényle) ; $R^2$ représente un groupe méthyle ou éthyle ;

$R^3$ représente un atome d'hydrogène, un atome de chlore ou un groupe méthyle.

Les groupes alkyles, alcoxy, acyles et acyloxy contenus dans les groupes représentés par $R^1$, $R^5$ et $R^6$ ou bien $R^4$, $R^7$, $R^8$, $R^9$, $R^{10}$ et Y de la formule générale I doivent contenir à chaque fois 1 à 4 atomes de carbone, et l'on préfère alors un groupe méthyle, éthyle, propyle, isopropyle, méthoxy, éthoxy, propoxy, isopropoxy, formyle, acétyle, propionyle et isopropionyle, procédé caractérisé en ce qu'on fait réagir un composé de formule générale II

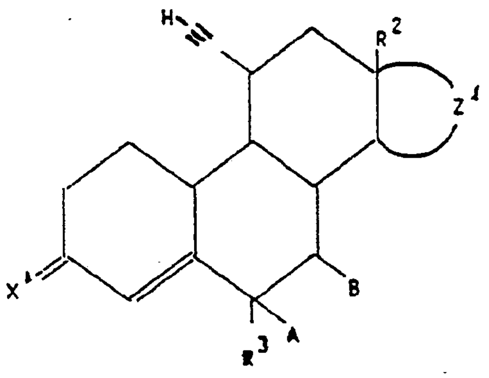

(II),

[dans laquelle A, B, $R^2$ et $R^3$ ont le sens indiqué ci-dessus ; $X^1$ représente un atome d'oxygène ou 2 atomes d'hydrogène, et Z' a le même sens que Z, mais des atomes d'hydrogène acétylénique éventuellement présents dans Z sont protégés dans Z' et des groupes O-acyles éventuellement présents dans Z sont présents dans Z' sous forme de groupes OH, et des groupes OH éventuellement présents dans Z ou Z' sont éventuellement protégés] avec un halogénure de formule générale III

$$R^{1'}\text{-}V \quad (III)$$

dans laquelle $R^{1'}$ a le même sens que $R^1$ mais des groupes O-acyles éventuellement présents dans $R^1$ sont présents dans $R^{1'}$ sous forme de groupes OH et les groupes OH éventuellement présents dans $R^1$ ou $R^{1'}$ sont éventuellement protégés, et V représente un atome de chlore, de brome ou d'iode,

à 0 jusqu'à 100°C dans un solvant polaire en faisant agir un agent de copulation, on enlève du produit ainsi obtenu les groupes protecteurs d'acétylènes terminaux éventuellement présents, on élimine éventuellement les groupes protecteurs de OH et, si on le souhaite, on estérifie ou éthérifie les groupes hydroxyles présents dans $R^1$, Y et Z, en formant le produit de formule générale I dans laquelle X a le sens de X' et, éventuellement ensuite, on fait réagir avec du chlorhydrate d'hydroxylamine en présence d'amines tertiaires, à des températures comprises entre –20°C et +40°C, pour obtenir un produit de formule générale I dans laquelle X représente le groupement hydroxy imino N ~OH.

7. Préparations pharmaceutiques, caractérisées en ce qu'elles contiennent des composés selon les revendications 1 à 5.

8. Utilisation des composés selon les revendications 1 à 5 pour préparer des médicaments.